# EUROPÄISCHE PATENTANMELDUNG

(11) **EP 1 484 004 A1**
(43) Veröffentlichungstag der Anmeldung: **08.12.2004**
(21) Anmeldenummer: 04405323.9
(22) Anmeldetag: 26.05.2004
(51) Int. Cl.: A61B 1/247

(54) **Inspektions-Spiegelchen für Zahnärzte und Chirurgen**

(30) Priorität: 06.06.2003 CH 10042003
(71) Anmelder: Erbicol SA, 6828 Balerna (CH)
(72) Erfinder: Romelli, Luca, 6926 Montagnola (CH); Romelli, Massimo, 1246 Corsier./GE (CH)
(74) Vertreter: Gaggini, Carlo

(57) **Zusammenfassung**

Die vorliegenden Erfindung betrifft ein Inspektions-Spiegelchen für Zahnärzte und Chirurgen, das widerstandsfähig ist gegen Abscheuern, gegen chemische Waschmittel und gegen Sterilisierung im Autoklav.

Das Spiegelchen besteht aus einem länglichen Haltestiel (1 ), der an einem seiner Enden ein reflektierendes Element (3) trägt, das aus durchsichtigem weissem Saphir besteht, auf dessen einer Seite ein reflektierender metallischer Überzug (6) angebracht ist.

Die vorliegenden Erfindung ist dadurch gekennzeichnet, dass der Haltstiel (1) aus einem einzigen Stück gefertigt ist, das aus synthetischem Material besteht, welches chemische und mechanische Eigenschaften besitzt, die den genannten Anforderungen genügen, und der an einem seiner Enden eine ebene Fläche (2) aufweist, auf der das reflektierende Element mittels Verklebung befestigt wird. Dank der Tatsache, dass der Haltestiel (1) als "Monoblock" als ein einziges Stück ausgebildet ist, entfallen alle Verbindungsstellen zwischen zwei oder mehreren Teilen der gemäss dem Stand der Technik bekannten Haltestiele, so dass jede Gefahr ausgeschaltet ist dass sich pathogene Keime in den Spalten festsetzen und infolge dieser Verunreinigungen die nächsten Patienten infizieren.

Die Vorteile der vorliegenden Erfindung sind hauptsächlich wirtschaftlicher Natur - dank geringeren Herstellkosten - und hygienischer Natur.

## Beschreibung

Die vorliegende Erfindung betrifft ein Inspektions-Spiegelchen für Zahnärzte und Chirurgen, das Widerstand gegen Abrasion bzw. Abscheuem, gegen chemische Waschmittel und Sterilisieren im Autoklav bietet, gemäss dem Oberbegriff des Anspruchs 1.

In jüngster Zeit waren wichtige qualitative Verbesserungen an Inspektions-Spiegelchen, vorab solchen für Zahnärzte, zu verzeichnen. Die neuen Arbeitsmethoden, bei welchen anstelle der herkömmlichen Bohrer Abrasionssysteme zum Einsatz kommen, die mit Luftstrom und abrasivem Granulat-Material der Art von Aluminium-Oxyd arbeiten, haben den grossen Vorteil, dass der Eingriff oft ohne Anwendung von Anästhesie-Mitteln beim Patienten erfolgen kann. Die herkömmlichen Zahnspiegelchen wiesen jedoch den Nachteil auf, rasch von diesen abrasiven Partikeln zerkratzt zu werden, so dass sie nach einmaligem Einsatz unbrauchbar wurden. Dies erwies sich als sehr teuer. Zum Vermeiden dieses Nachteils wurden Inspektions-Spiegelchen vorgeschlagen, bei welchen die normalen reflektierenden Spiegelchen aus Glas durch solche ersetzt wurden, die aus durchsichtigem weissem Saphir hergestellt sind, und auf deren einen Seite eine reflektierende Metallschicht aufgebracht wurde. Der Saphir ist so hart, dass er praktisch nur von Diamant zerkratzt werden kann, so dass auch die Verwendung härtester Abrasivpulver die Oberfläche des Spiegelchens nicht angreifen können.

Ein Beispiel eines derartigen Spiegelchens ist in der PCT-WO-00138586 gezeigt, in der ein Spiegelchen beschrieben ist, das mit einem Haltestiel und einem gerundeten Rahmen versehen ist, der das reflektierende Spiegelchen trägt und mit dem Haltestiel fest mittels Verlötung oder auf ähnliche Weise verbunden bleibt. Der Haltestiel selbst besteht aus zwei aufeinander verschraubten Teilstücken. Dieses Spiegelchen genügt den Anforderungen an die Abriebfestigkeit sowie der Resistenz gegen chemische Waschmittel und gegen die Sterilisierung im Autoklav vollauf, weist jedoch noch zwei bedeutende Nachteile auf, nämlich dass die Herstellung viel zu teuer ist, da es aus verschiedenen Metallteilen (beispielsweise aus rostfreiem Stahl) besteht, die durch Verlöten oder Verschweissen und/oder mechanisch, beispielsweise durch Verschrauben, zusammenmontiert werden müssen, und dass keine absolute hygienische Sicherheit gewährleistet ist. Zwischen den zusammengesetzten Teile verbleiben Spalten, wenn auch sehr kleine, in welchen sich pathogene Keime einnisten können, die manchmal Sterilisierungs-Massnahmen unbeschadet überleben können. Heute, in Anbetracht der immer grösseren Gefahren der Infektion mit Krankheiten, sogar unheilbaren, ist daher von vorrangiger Wichtigkeit, dass jede Infektionsgefahr durch Übertragung von Blut und/oder Speichel ausgeschlossen wird, dass also bei der Herstellung von wiederverwendbaren Spiegelchen grösste Sicherheit für absolute Hygiene gewährleistet werden muss.

Auch andere Vorschläge für Inspektions-Spiegelchen sind bekannt (siehe beispielsweise die US-5'906'487 und US-5'269'683) und zeigen die gleichen Probleme: Alle zielen darauf ab, beste Bedingungen für das Reflektieren des Bildes zu schaffen, auch mit Vergrösserung des Bildes, und für die Widerstandsfähigkeit gegen mechanische Beanspruchung und gegen die Einflüsse von Waschprozessen, messen jedoch weder dem Hygieneaspekt noch den Herstellkosten besondere Bedeutung zu.

Auf diesem Gebiet zeigen sich die besondern Vorteile der vorliegenden Erfindung, deren Zweck darin besteht, eine optimale Lösung für Inspektions-Spiegelchen für Zahnärzte zu schaffen, aber auch allgemein für Chirurgen, die nicht nur kostengünstig ist, sondern auch hygienisch einwandfrei ist, und die dauerhaft und unzerkratzbar ist, mechanisch widerstandsfähig und resistent gegen Waschmittel und Behandlung im Autoklav, wie sie von den bisherigen Lösungen bekannt sind, insbesondere aus der PCT-WO-00/38568.

Diese Zielsetzungen werden erfüllt dank den Eigenschaften gemäss dem kennzeichnenden Teil des Anspruchs 1.

Die Verwendung eines einteiligen Haltestiels aus synthetischem Material, das den verschiedenen Einflüssen widersteht, in welchem das reflektierende Element durch Einkleben befestigt wird, ermöglicht die Herstellung des Haltestiels nach dem Druckgussverfahren, und das Vermeiden jeglicher Spaltbildung zwischen Einzelteilen. Auf diese Weise werden zwei mit der vorliegenden Erfindung verbundene, grundlegende Vorteile gewonnen: Tiefe Herstellkosten, da nichts weniger kostet als die Massenherstellung von Präzisionsteilen aus Kunststoff nach dem Pressgussverfahren, und die Hygiene dank des Fehlens irgendwelcher verborgener Ritzen für Keime. Der einzige Punkt, der bei der Herstellung des erfindungsgemässen Spiegelchens besondere Beachtung erheischt, ist die Ausbildung einer Übergangszone zwischen dem reflektierenden Element und dem Trägerblock aus Kunststoff ohne Risse oder Ritzen, was mit Hilfe bekannter Poliermittel erreicht werden kann, wie dies im Folgenden besonders erklärt wird.

Die Ansprüche 2 bis 9 betreffen sodann bevorzugte Ausführungsformen der vorliegenden Erfindung, die im Folgenden unter Bezugnahme auf die beigefügten Abbildungen detailliert erklärt werden. Die Abbildungen zeigen in der:
- Fig. 1: Eine perspektivische Darstellung eines erfindungsgemässen Spiegelchens,
- Fig. 2: Das erfindungsgemässe Spiegelchen gemäss der Fig. 1, von oben gesehen,
- Fig. 3: Das erfindungsgemässe Spiegelchen gemäss der Fig. 1, in einer Seitenansicht gesehen,
- Fig. 4: Ein Konstruktions-Detail der Befestigungszone des reflektierenden Elementes, und in der
- Fig. 5: Eine Ausführungsvariante der vorliegenden Erfindung mit doppeltem reflektierendem Element.

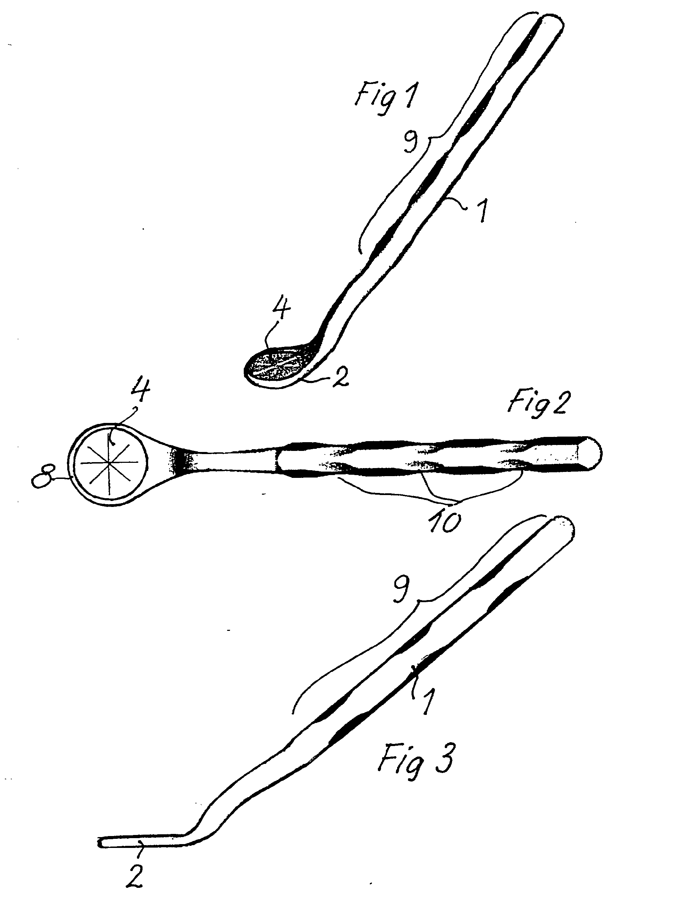

In der Fig. 1 ist der mit 1 bezeichnete Haltestiel eines erfindungsgemässen Zahnspiegelchens in perspektivischer Ansicht dargestellt. An seinem einen Ende bildet der Haltestiel eine ebene Fläche 2, auf der das reflektierende Element 3 mittels Aufkleben befestigt wird.

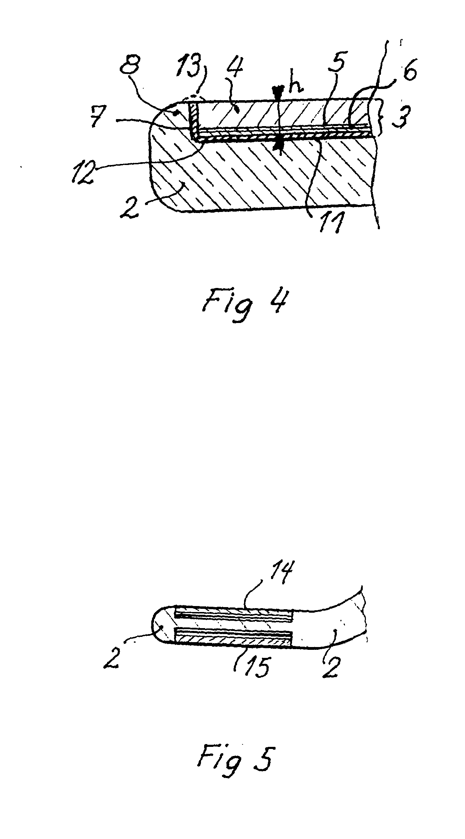

Das reflektierende Element 3 besteht, wie dies aus den oben genannten Dokumenten an sich bekannt ist, aus einer Scheibe aus transparentem synthetischen weissen Saphir 4 (was im Schnitt gemäss der Fig. 4 besser ersichtlich ist, die eine Detailansicht im Querschnitt durch die ebene Fläche 2 zeigt, welche das reflektierende Element 3 trägt), auf deren einen Seite, nämlich auf der mit 5 bezeichneten Seite, mit einem metallischen reflektierenden Überzug 6 versehen ist. Die Dicke des metallischen Überzuges 6 ist in Wirklichkeit äusserst gering und ist in den Figuren 4 und 5 zur besseren Erkennbarkeit stark vergrössert gezeichnet. In Wirklichkeit liegt diese Dicke in der Grössenordnung von wenigen Tausendstelmillimetem. Gemäss einer bevorzugten Ausführungsform der vorliegenden Erfindung besteht der reflektierende metallische Überzug 6 aus Aluminium oder aus Silber.

Gemäss der vorliegenden Erfindung ist sodann vorgesehen, dass das reflektierende Element 3 auf der ebenen Fläche 2 des Haltestiels 1 befestigt ist, mittels Verklebung an der ebenen Seite 5, auf welcher der metallische Überzug 6 aufgebracht ist. Die Form des Haltestiels 1 kann entsprechend dem Anwendungszweck, für den das Spiegelchen ausgelegt ist, verschieden ausgebildet sein. Für ein Inspektions-Spiegelchen für Zahnärzte wird der Haltestiel in solcher Weise ausgebildet, dass die ebene Fläche 2 mit dem Schaft des Haltestiels einen Winkel von etwa 30° bildet, wie dies von den normalen Zahnarzt-Spiegeln her bekannt ist. Wichtig ist im Rahmen der vorliegenden Erfindung einzig, dass der Haltestiel 1 zusammen mit der ebenen Fläche 2 als ein einziges Stück Pressguss erzeugt wird, das von Anfang an alle Eigenschaften des fertigen Spiegelchens besitzt. Der aus einem Stück bestehende Haltestiel 1 ist daher vorzugsweise bereits mit mindestens einer Ausnahme 7 versehen, in welche das reflektierende Element 3 in der nachfolgend zu beschreibenden Weise eingesetzt werden kann: Dies bedeutet, dass die ebene Oberfläche 2 einen Rand 8 aufweist, der die Ausnehmung 7 seitlich begrenzt, die von genau gleicher Grösse ist wie der Umfang des reflektierenden Elements 3.

Ferner ist gemäss einer bevorzugten Ausführungsform der vorliegenden Erfindung vorgesehen, dass der Haltestiel 1 in seiner Griffzone 9 eine Strukturierung 10 aufweist, welche die Form von Rippen, Rillen, Erhebungen, usw., hat, und welche bereits beim Pressgiessen des Stücks erzeugt wird.

Wie bereits weiter oben festgestellt wurde, ist besonders wichtig, dass beim Einbau des reflektierenden Elements 3 auf der ebenen Oberfläche 2 kein Grat und keine Spalten entstehen, in welchen sich Keime und Bakterien einnisten können.

In der Fig. 4 ist die bevorzugte Montageweise dargestellt, mittels welcher dieses Resultat erreicht wird. Gemäss dieser Montageweise entspricht die Höhe des Randes 8 im wesentlichen der Dicke h des reflektierenden Elements, so dass eine Fläche gebildet wird, auf der das reflektierende Element 3 in solcher Weise eingesetzt und verleimt wird, dass zwischen dem Rand 8 und dem Umfang des reflektierenden Elements 3 keinerlei Spalt frei bleibt.

In der Fig. 4 ist die mit 12 bezeichnete Klebstoff-Schicht, welche die Ausnehmung 7 auf ihrem Grund bedeckt und somit das reflektierende Element 3 auf der ebenen Fläche 2 festklebt und den Spalt zwischen dem Umfang des reflektierenden Elements 3 und dem Rand 8 vollständig verschliesst. In Wirklichkeit ist die Dicke dieser Klebstoff-Schicht 12 so klein als möglich gehalten, und sie ist praktisch nicht mehr sichtbar. In der Fig. 4 ist sie somit sehr stark über die erforderliche Dicke hinaus vergrössert gezeichnet, insbesondere zwischen dem reflektierenden Element 3 und dem Rand 8. Dabei ist selbstverständlich, dass versucht wird, ein möglichst kleines Spiel zwischen dem reflektierenden Element 3 und dem Rand 8 zu erreichen, das nachher vom Klebstoff ausgefüllt wird. Für bestmögliches Verschliessen des Spaltes zwischen dem Rand 8 und dem reflektierenden Element 3 wird beim Verkleben des reflektierenden Elements 3 in der Ausnehmung 7 darauf geachtet, dass ein bisschen mehr Klebstoff, als zum Auffüllen der Zwischenräume erforderlich ist, eingebracht wird, in solcher Weise dass, wenn das reflektierende Element 3 in die Ausnehmung 7 gedrückt wird, bis die obere Oberfläche des reflektierenden Elements 3 (d.h. die durchsichtige Oberfläche des Saphirs) genau auf die gleiche Höhe zu liegen kommt wie der Rand 8, etwas Klebstoff aus dem "vertikalen" Spalt zwischen dem reflektierenden Element 3 und dem Rand 8 austritt und einen kleinen Klebstoffwulst (in der Fig. 4 gestrichelt dargestellt und mit 13 bezeichnet) bildet, der in einem nachfolgenden Bearbeitungsschritt genau entfernt wird. Bei diesem Arbeitsschritt wird die Gefahr ausgeschlossen, dass sich auch nur der kleinste Spalt zwischen dem Rand 8 und dem reflektierenden Element 3 bildet, womit einer der grundlegenden Vorteile der vorliegenden Erfindung erreicht wird.

Wenn sichergestellt ist, dass der Rand 8 im wesentlichen die gleiche Höhe aufweist wie das reflektierende Element 3, kann gesagt werden, dass die Dicke der Klebstoffschicht 12 zwar vernachlässigbar klein ist, wird ihr dennoch beim Festlegen der Tiefe der Ausnehmung 7 gegenüber der Dicke des reflektierenden Elements 3 Rechnung getragen.

Gemäss einer anderen, in der Fig. 5 dargestellten, bevorzugten Ausführungsform der vorliegenden Erfindung, ist vorgesehen, dass der Haltestiel 1 mit zwei reflektierenden Elementen 14 und 15 bestückt ist, die auf je einer Seite der ebenen Fläche 2 angebracht sind. Der Vorteil dieser Ausführungsform ist evident: Die Beobachtungs- und Inspektionsmöglichkeiten in der Mundhöhle oder in anderen Bereichen, die zu überprüfen sind, werden dadurch erweitert.

Gemäss einer weiteren bevorzugten Ausführungsform der vorliegenden Erfindung ist sodann vorgesehen, dass zum Befestigen des reflektierenden Elementes 2, 14 oder 15 auf der ebenen Fläche 2 des Haltestiels 1 mittels Verklebung durchsichtiges Silikon verwendet wird.

Eine sonders vorteilhafte Lösung für die vorliegende Erfindung besteht darin, dass das reflektierende Element 3 die Form einer kreisrunden Scheibe aufweist, deren Durchmesser zwischen 6 und 30 mm liegt. Die kreisrunde Form ist naturgemäss am einfachsten herzustellen und ist damit die vorteilhafteste Form, die auch bei konventionellen Spiegelchen am häufigsten Verwendung findet, wobei innerhalb der genannten Grenzen der ideale Durchmesser vom vorgesehenen Anwendungszweck des Inspektions-Spiegelchens abhängt.

Ferner ist die Möglichkeit vorgesehen, dass das erfindungsgemässe Inspektions-Spiegelchen ein konkav ausgebildetes reflektierendes Element 3 trägt, mit dem eine Vergrösserung des reflektierten Bildes ermöglicht wird. Auch diese Ausbildungsform ist bei den konventionellen Spiegelchen bekannt und ist beispielsweise in der bereits vorher erwähnten US-5'906'487 beschrieben; diese Ausbildungsform ist jedoch neu im Zusammenhang mit einem aus einem Stück bestehenden "Monoblock" Inspektions-Spiegelchen wie es in der vorliegenden Anmeldung beansprucht wird.

Gemäss einer weiteren Ausführungsform der vorliegenden Erfindung ist sodann vorgesehen, dass das Material, aus dem der Haltestiel 1 des Spiegelchens gefertigt wird, ein Thermoplast-Kunststoff auf Basis eines halbkristallinen, partiell aromatischen Kopolyamids ist. Ein solches Material ist im Handel erhältlich von der Firma Emserwerke in DomatIEms (Schweiz) unter dem Handelsnamen Grivory HTV-4X1 Natural. Dieses Material erwies sich als sehr geeignet, da es den Anforderungen, welche die Arbeitsbedingungen, die Widerstandsfähigkeit und die Kostenlage an das erfindungsgemässe Spiegelchen stellen, optimal genügt.

### Liste der in den Figuren verwendeten Bezugsziffern

- 1: Haltestiel
- 2: ebene Fläche
- 3: reflektierendes Element
- 4: Scheibe aus weissem Saphir
- 5: ebene Oberfläche der Saphir-Scheibe
- 6: metallischer Überzug
- 7: Ausnehmung
- 8: Rand
- 9: Griffbereich
- 10: Strukturierung
- 11: Oberfläche
- 12: Klebstoff-Schicht
- 13: Klebstoffwulst
- 14: reflektierendes Element
- 15: reflektierendes Element

## Patentansprüche

1. lnspektions-Spiegelchen für Zahnärzte und Chirurgen, das widerstandsfähig ist gegen Abscheuern und chemische Waschmittel, und das im Autoklav sterilisierbar ist, bestehend aus einem länglichen Haltestiel (1), der an seinem einen Ende mindestens ein reflektierendes Element (3) trägt, das aus einer Scheibe aus durchsichtigem synthetischem weissem Saphir (4) besteht, auf dessen einer Seitefläche (5) ein metallischer reflektierender Überzug (6) angebracht ist,
**dadurch gekennzeichnet, dass**
der Haltestiel (1) aus einem einzigen Stück besteht und aus einem synthetischen Material gefertigt ist, das widerstandsfähig ist gegen Abnützung, gegen mechanische Beanspruchungen, gegen chemische Waschmittel, und gegen die hohen Temperaturen, die in einem Sterilisier-Autoklaven herrschen, und an dessen einem Ende eine ebene Fläche (2) ausgebildet ist, auf der mittels Verklebung die Fläche (5) des reflektierenden Elements (3) befestigt wird, auf welcher der reflektierende metallische Überzug (6) angebracht ist.

2. Inspektions-Spiegelchen gemäss dem Anspruch 1,
**dadurch gekennzeichnet, dass**
die ebene Fläche (2) mit einem Rand (8) versehen ist, der im wesentlichen die gleiche Höhe aufweist, wie die Höhe (h) des reflektierenden Elements (3), so dass sie eine Oberfläche bildet, auf der das reflektierende Element (3) in solcher Weise aufgesetzt und verklebt wird, dass zwischen dem Rand (8) und dem Umfang des reflektierenden Elements (3) keinerlei Spalt frei bleibt.

3. Inspektions-Spiegelchen gemäss dem Anspruch 1 und/oder 2,
**dadurch gekennzeichnet, dass**
der Haltestiel (1) zwei reflektierende Elemente (14, 15) trägt, die auf je einer Seite seiner ebenen Fläche (2) befestigt sind.

4. Inspektions-Spiegelchen gemäss einem der Ansprüche 1 bis 3,
**dadurch gekennzeichnet, dass**
das reflektierende Element (3; 14, 15) auf der ebenen Fläche (2) des Haltestiels (1) mittels Verklebung mit durchsichtigem Silikon befestigt ist.

5. Inspektions-Spiegelchen gemäss dem Anspruch 1,
**dadurch gekennzeichnet, dass**
der Haltestiel (1) im Griffbereich (9) des Benützers eine Strukturierung (10) in Form von Rippen, Rillen, Erhebungen, usw., aufweist, welche das Festhalten erleichtert.

6. Inspektions-Spiegelchen gemäss dem Anspruch 1,
**dadurch gekennzeichnet, dass**
der Haltestiel (1) aus thermoplastischem Material auf Basis eines halbkristallinen oder partiell aromatischen Kopolyamids gefertigt ist.

7. Inspektions-Spiegelchen gemäss dem Anspruch 1,
**dadurch gekennzeichnet, dass**
der reflektierende metallische Überzug 6) des reflektierenden Elements (3) aus Aluminium oder aus Silber besteht.

8. Inspektions-Spiegelchen gemäss dem Anspruch 1,
**dadurch gekennzeichnet, dass**
das reflektierende Element (3) eine kreisrunde Scheibe ist, deren Durchmesser zwischen 6 und 30 mm liegt.

9. Inspektions-Spiegelchen gemäss dem Anspruch 1,
**dadurch gekennzeichnet, dass**
das reflektierende Element (3) konkav ausgebildet ist, so dass eine Vergrösserung der reflektierten Bilder erreicht wird.
